# EUROPEAN PATENT SPECIFICATION

(11) **EP 1 965 783 B1**
(45) Date of publication and mention of the grant of the patent: **19.06.2013**
(21) Application number: 06820700.0
(22) Date of filing: 22.12.2006
(51) Int. Cl.: A61K 31/155, A61K 31/426, A61K 31/4439, A61P 3/10

(54) **TREATMENT OF TYPE 2 DIABETES WITH A COMBINATION OF DPIV INHIBITOR AND METFORMIN OR THIAZOLIDINEDIONE**
BEHANDLUNG VON DIABETES TYP 2 MIT EINER KOMBINATION AUS DPIV-HEMMER UND METFORMIN ODER THIAZOLIDINDION
TRAITEMENT DU DIABETE DE TYPE 2 PAR COMBINAISON D'UN INHIBITEUR DE DPIV A DE LA METFORMINE OU DE LA THIAZOLIDINEDIONE

(30) Priority: 23.12.2005 GB 0526291
(43) Date of publication of application: 10.09.2008
(73) Proprietor: Royalty Pharma Collection Trust, Wilmington DE 19890 (US)
(72) Inventor: RACHMAN, Jonathan, Oxford Oxfordshire OX4 6LT (GB)
(74) Representative: Mintz Levin Cohn Ferris Glovsky and Popeo LLP
(86) International application number: PCT/GB2006/050475
(87) International publication number: WO 2007/072083

(56) References cited:
- WO-A-00/53171
- WO-A-2005/020983
- SUZUKI M ET AL: "EFFECT OF PIOGLITAZONE IN COMBINATION WITH METFORMIN ON DIABETES AND OBESITY IN WISTAR FATTY RATS" DIABETES, NEW YORK, NY, US, vol. 48, no. SUPPL 1, 19 June 1999 (1999-06-19), page A454, XP001065260 ISSN: 0012-1797
- ANONYMOUS: "Sitagliptin (MK-0431) for Type 2 Diabetes shows promise in Phase II clinical trials"[Online] 11 June 2005 (2005-06-11), XP002424688 Retrieved from the Internet: URL:http://www.medicalnewstoday.com/medica lnews.php?newsid=25962> [retrieved on 2007-03-14]
- ANONYMOUS: "New Phase III Data Showed Significant Glucose-Lowering Efficacy of JANUVIA(TM), an Investigational Medicine for Type 2 Diabetes, When Taken Simultaneously with Metformin"[Online] 14 September 2006 (2006-09-14), XP002424689 Retrieved from the Internet: URL:http://209.18.123.244/news/bnitem.aspx ?name=5479835> [retrieved on 2007-03-14]
- ANONYMOUS: "Highlights of Prescribing Information: JANUVIA Tablets"[Online] October 2006 (2006-10), XP002424690 Retrieved from the Internet: URL:http://www.merck.com/product/usa/pi_ci rculars/j/januvia/januvia_pi.pdf> [retrieved on 2007-03-14]
- HINKE S A ET AL: "Metformin effects on dipeptidylpeptidase IV degradation of glucagon-like peptide-1", BIOCHEMICAL AND BIOPHYSICAL RESEARCH COMMUNICATIONS, ACADEMIC PRESS INC. ORLANDO, FL, US, vol. 291, no. 5, 15 March 2002 (2002-03-15), pages 1302-1308, XP002976338, ISSN: 0006-291X, DOI: 10.1006/BBRC.2002.6607
- YASUDA N ET AL: "Enhanced secretion of glucagon-like peptide 1 by biguanide compounds", BIOCHEMICAL AND BIOPHYSICAL RESEARCH COMMUNICATIONS, ACADEMIC PRESS INC. ORLANDO, FL, US, vol. 298, no. 5, 15 November 2002 (2002-11-15), pages 779-784, XP002976340, ISSN: 0006-291X, DOI: 10.1016/S0006-291X(02)02565-2
- KRENTZ ANDREW J ET AL: "ORAL ANTIDIABETIC AGENTS - CURRENT ROLE IN TYPE 2 DIABETES MELLITUS", DRUGS : INTERNAT. JOURNAL OF CURRENT THERAPEUTICS AND APPLIED PHARMACOLOGY REVIEWS, FEATURING EVALUATIONS ON NEW DRUGS AND DRUG THERAPY, AND DRUG LITERATURE ABSTRACTS, WOLTERS KLUWER HEALTH ADIS, AUSTRALIA, vol. 65, no. 3, 1 January 2005 (2005-01-01), pages 385-411, XP009085446, ISSN: 0012-6667, DOI: 10.2165/00003495-200565030-00005
- TURNER R C ET AL: "GLYCEMIC CONTROL WITH DIET, SULFONYLUREA, METFORMIN, OR INSULIN IN PATIENTS WITH TYPE 2 DIABETES MELLITUS PROGRESSIVE REQUIREMENT FOR MULTIPLE THERAPIES (UKPDS 49)", JAMA THE JOURNAL OF THE AMERICAN MEDICAL ASSOCIATION, AMERICAN MEDICAL ASSOCIATION, US, vol. 281, no. 21, 2 June 1999 (1999-06-02) , pages 2005-2012, XP008011494, ISSN: 0098-7484, DOI: 10.1001/JAMA.281.21.2005

## Description

### FIELD OF THE INVENTION

This invention relates to a combination of a DPIV inhibitor and metformin or thiazolidinedione for use in a therapeutic method for glycaemic control, in particular a method for the treatment of type 2 diabetes.

### BACKGROUND ART

Diabetes mellitus is a chronic metabolic disorder characterized by the presence of hyperglycaemia (raised blood glucose concentrations). It may be divided into four general subclasses, including i) type 1 or insulin-dependent diabetes mellitus (IDDM) (caused by beta-cell destruction and characterized by absolute insulin deficiency), ii) type 2 or non-insulin-dependent diabetes mellitus (NIDDM) (characterized by impaired insulin action and impaired insulin secretion), iii) other specific types of diabetes (associated with various identifiable clinical conditions or syndromes such as genetic defects of beta-cell function e.g. maturity-onset diabetes of the young types 1 - 3 and point mutations in mitochondrial DNA), and iv) gestational diabetes mellitus.

The prevalence of type 2 diabetes is high and is growing at an alarming rate. The global burden of diabetes mellitus is expected to reach 300 million by the year 2025, with more than 90% of these individuals having type 2 diabetes.

The predominant pathophysiological defects leading to hyperglycaemia in type 2 diabetes are impaired insulin action (insulin resistance) and impaired insulin secretion (beta-cell dysfunction). Treating hyperglycaemia is therapeutically important in diabetes mellitus in order to prevent symptoms caused by the raised blood glucose concentrations, such as polyuria (excessive urination) and polydipsia (excessive thirst), and to reduce the risk of diabetic complications. The chronic hyperglycaemia of diabetes mellitus is associated with significant, often devastating long-term complications in the eyes, kidneys, nerves and blood vessels. The largest study of pharmacotherapy in type 2 diabetes, The United Kingdom Prospective Diabetes Study (UKPDS), demonstrated that lowering blood glucose concentrations with pharmacotherapy in type 2 diabetes reduces the risk of complications. [*Lancet* **352**:837-853, 1998]. The study showed that there was no lower threshold for the benefits of glucose lowering and that any additional glucose lowering would further reduce the risk of development of diabetic complications.

The UKPDS also demonstrated that an inexorable decline in beta-cell function occurs with time in type 2 diabetes [Diabetes 44:1249-1258, 1995]. This leads, in the majority of patients, to worsening of glycaemic control with time, requiring addition of more and more therapies as the disease progresses.

There are a number of oral agents currently available to treat type 2 diabetes. The three classes of agent which are most commonly prescribed are metformin, the sulphonylureas and the PPARγ agonist thiazolidinediones (TZDs). Metformin acts by decreasing glucose output from the liver, it is associated with gastrointestinal side-effects in many patients and has no impact on the decline in beta-cell function with time. The sulphonylureas act by increasing insulin secretion, are associated with the side effects of weight gain and hypoglycaemia (low blood glucose concentrations) and, like metformin, have no impact on the decline in beta-cell function with time (see UKPDS). The TZDs act as insulin sensitizers and, whilst they are the only class of oral agent currently licensed to treat type 2 diabetes which decreases the rate of decline of beta-cell function with time, they are associated with the side effects of weight gain and oedema.

It is known that DPIV inhibitors may be useful for the treatment of impaired glucose tolerance and diabetes mellitus, see International Patent Application No. WO97/40832. Clinical data suggest that the glucose lowering that occurs in response to treatment with DPIV inhibitors is not accompanied by significant side effects such as weight gain and hypoglycaemia. In addition, pre-clinical data exist which suggest that DPIV inhibitors preserve beta-cell mass, see International Patent Application No. WO01/72290, raising the possibility that treatment with a DPIV inhibitor would decrease the rate of decline of beta-cell function that occurs with time in type 2 diabetes.

The current treatment paradigm in type 2 diabetes is to commence therapy with one class of oral agent, following 'failure' of diet and exercise, with addition of a second (and later a third) class of agent only when glycaemic control is no longer adequate on the initial agent(s), i.e. the treatment comprises first-line monotherapy followed by second-line combination therapy followed by third-line combination therapy. Thus, for example, the DPIV inhibitor sitagliptin phosphate (Januvia™) is approved for use in the US in combination with metformin or a PPARγ agonist only when the metformin or PPARγ agonist alone, with diet and exercise, does not provide adequate glycaemic control. Currently, no DPIV inhibitors are licensed for simultaneous commencement of therapy with a second class of anti-diabetic agent on initiation of oral therapy, after 'failure' of diet and exercise, in type 2 diabetes.

There is a need to find new and improved regimens for the treatment of type 2 diabetes. The present invention provides a novel first line, i.e. initial, combination therapy for the treatment of type 2 diabetes i.e. for the treatment of type 2 diabetes after 'failure' of diet and exercise. This method has the potential to provide greater efficacy than current monotherapy given alone without introducing any side-effect liability.

### SUMMARY OF THE INVENTION

The present invention provides a DPIV inhibitor in combination with metformin or a thiazolidinedione as first-line combination therapy for treatment of type 2 diabetes, i.e. to be used in patients who do not have adequate glycaemic control on treatment with diet and exercise alone.

### DETAILED DESCRIPTION OF THE INVENTION

A method for the treatment of type 2 diabetes in a human, comprises administering a combination of a DPIV inhibitor and metformin or a thiazolidinedione as first-line therapy, to a human in need thereof.

First-line therapy is defined as the first course of pharmaceutical treatment used against a disease, thus in the present case it represents the first pharmacological intervention to treat type 2 diabetes in a patient diagnosed as having inadequate glycaemic control. In a type 2 diabetic patient this will generally be a patient whose hyperglycaemia can no longer be controlled satisfactorily by diet, weight reduction and/or exercise alone. The method may be used to treat a human that has failed to achieve adequate glycaemic control using diet, weight reduction and/or exercise alone. Such a patient population has not previously been treated with a first line combination therapy comprising a DPIV inhibitor and metformin or a thiazolidinedione. Type 2 diabetes is thereby treated.

A method for the treatment of type 2 diabetes in a human, which mammal has not previously been treated with an antidiabetic agent, e.g. metformin or a thiazolidinedione alone, comprises administering a combination of a DPIV inhibitor and metformin or a thiazolidinedione to said human.

The invention therefore also provides a DPIV inhibitor in combination with metformin or a thiazolidinedione for the treatment of type 2 diabetes in a human, which human has not previously been treated with an antidiabetic agent, e.g. metformin or a thiazolidinedione alone.

The invention represents a fundamental paradigm shift away from the traditional approach of only adding additional pharmacological therapy, i.e. a second therapeutic agent, when the initial pharmacological therapy is considered to be 'failing', i.e. not providing adequate glycaemic control in the patient.

The invention provides significant advantages over traditional therapies for glycaemic control, including the possibility for:
greater blood glucose lowering than with monotherapy e.g. using metformin or a TZD alone, this is advantageous because there is no lower threshold for the benefits of glucose lowering and current agents have modest efficacy;
targeting of the two main pathophysiological defects in type 2 diabetes (insulin resistance and beta-cell dysfunction) simultaneously;
maximisation of the opportunity for early positioning in the treatment continuum of a therapy which reduces decline in beta-cell function; and/or
reduced risk of hypoglycaemia or weight gain.

Coadministration of the DPIV inhibitor and metformin or a thiazolidinedione includes administration of a formulation which includes both the DPIV inhibitor and metformin or a thiazolidinedione, or the essentially simultaneous, sequential or separate administration of separate formulations of the DPIV inhibitor and metformin or a thiazolidinedione.

The DPIV inhibitor is preferably administered in combination with metformin. As referred to in the present application "metformin" includes any pharmaceutically acceptable salt of metformin e.g. the hydrochloride salt.

As referred to in the present application "thiazolidinedione" and the specific examples of thiazolidinediones referred to below includes any pharmaceutically acceptable salts of thiazolidinediones.

Suitable TZDs for use in accordance with the invention include (+)-5-[[4-[(3,4-dihydro-6-hydroxy-2,5,7,8-tetramethyl-2H-1-benzopyran-2-yl)methoxy]phenyl]methyl]-2,4-thiazolidinedione (troglitazone), 5-[4-[(1-methylcyclohexyl)methoxy]benzyl]thiazolidine-2,4-dione (ciglitazone), 5-[4-[2-(5-ethylpyridin-2-yl)ethoxy]benzyl]thiazolidine-2,4-dione (pioglitazone), 5-[(2-benzyl-2,3-dihydrobenzopyran)-5-ylmethyl)thiazolidine-2,4-dione (englitazone) and 5-[4-[2-(N-methyl-N-(2-pyridyl)amino)ethoxy]benzyl]thiazolidine-2,4-dione (rosiglitazone) and pharmaceutically acceptable salts thereof.

Preferred TZDs are 5-[4-[2-(5-ethylpyridin-2-yl)ethoxy]benzyl]thiazolidine-2,4-dione (pioglitazone), (+)-5-[[4-[(3,4-dihydro-6-hydroxy-2,5,7,8-tetramethyl-2H-1-benzopyran-2-yl)methoxy]phenyl]methyl]-2,4-thiazolidinedione (troglitazone) and 5-[4-[2-(N-methyl-N-(2-pyridyl)amino)ethoxy]benzyl]thiazolidine-2,4-dione (rosiglitazone) and salts thereof. A particularly preferred TZD is 5-[4-[2-(N-methyl-N-(2-pyridyl)amino)ethoxy]benzyl]thiazolidine-2,4-dione (rosiglitazone) and pharmaceutically acceptable salts thereof.

DPIV inhibitors bind to DPIV and inhibit DPIV enzyme activity. The DPIV inhibitor for use in the method of the invention is preferably a small molecule DPIV inhibitor. As referred to in the present application "DPIV inhibitor" includes any pharmaceutically acceptable salts of DPIV inhibitors.

Examples of DPIV inhibitors include compounds disclosed in the following patent applications: WO95/15309, WO95/29691, WO98/18763, WO98/19998, WO99/25719, WO99/38501, WO99/46272, WO99/61431, WO99/62914, WO99/67278, WO99/67279, WO00/34241, WO01/34594, WO01/40180, WO01/55105, WO01/52825, WO01/68603, WO01/72290, WO01/81304, WO01/81337, WO01/96295, WO01/97808, WO02/02560, WO02/08090, WO02/14271, WO02/30890, WO02/30891, WO02/38541, WO02/51836, WO02/53548, WO02/62764, WO02/66627, WO02/67918, WO02/68420, WO02/76450, WO02/83109, WO02/83128, WO03/00181, WO03/00181, WO03/00250, WO03/02530, WO03/02531, WO03/02553, WO03/02942, WO03/03250, WO03/03727, WO03/04496, WO03/04498, WO03/104229, WO03/24965, WO03/35057, WO03/35067, WO03/04498, WO03/33524, WO03/33671, WO03/37327, WO03/55881, WO03/57144, WO03/57200, WO03/57666, WO03/68748, WO03/68757, WO03/74500, WO03/84940, WO03/92605, WO03/101449, WO03/101958, WO03/106456, WO04/16587, WO04/16840, WO04/18468, WO04/18469, WO04/24184, WO04/26822, WO04/33455, WO04/37169, WO04/37181, WO04/41795, WO04/43940, WO04/46106, WO04/46148, WO04/48352, WO04/50022, WO04/50658, WO04/52850, WO04/103276, WO04/112701, WO05/12249, WO05/12308, WO05/12312, WO05/16911, WO05/19168, WO05/25554, WO05/26148, WO05/30751, WO05/37779, WO05/37828, WO05/40095, WO05/42533, WO05/42488, WO05/44195, WO05/47297, WO05/51949, WO05/51950, WO05/56541, WO05/56013, WO05/56003, WO05/58849, WO05/58901, WO05/63750, WO05/73186, WO05/75421, WO05/75426, WO05/77900, WO05/82906, WO05/82847, WO05/82349, WO05/82348, WO05/85246, WO05/87774, WO05/87235, WO05/95343, WO05/95381, WO05/95339, WO05/97798, WO05/100334, WO05/108382, WO05/113510, WO05/116012, WO05/11614, WO05/11629, WO05/118555, WO05/120494, EP1245568, EP1258476, EP1258480, EP1338595, JP2002265439 and JP2003300977.

Examples of specific DPIV inhibitors include sitagliptin, vildagliptin, saxagliptin, denagliptin and alogliptin and salts thereof.

A preferred DPIV inhibitor for use in the method of the invention is glutaminyl thiazolidine or a pharmaceutically acceptable salt thereof, e.g. the hydrochloride salt, see WO03/072556.

Preferred combinations include the following:
glutaminyl thiazolidine, or a pharmaceutically acceptable salt thereof, and metformin;
glutaminyl thiazolidine, or a pharmaceutically acceptable salt thereof, and a thiazolidinedione;
glutaminyl thiazolidine, or a pharmaceutically acceptable salt thereof, and troglitazone, ciglitazone, pioglitazone, englitazone or rosiglitazone; and
glutaminyl thiazolidine, or a pharmaceutically acceptable salt thereof, and rosiglitazone.

Preferred embodiments of the invention thus include:
- a combination of glutaminyl thiazolidine or a pharmaceutically acceptable salt thereof and metformin as first-line therapy for administration to a human who has failed to achieve adequate glycaemic control using diet, weight reduction and/or exercise alone for the treatment of type 2 diabetes.
- a combination of glutaminyl thiazolidine or a pharmaceutically acceptable salt thereof and a thiazolidinedione as first-line therapy for administration to a human who has failed to achieve adequate glycaemic control using diet, weight reduction and/or exercise alone for the treatment of type 2 diabetes.
- a combination of glutaminyl thiazolidine or a pharmaceutically acceptable salt thereof, and metformin for administration to a human who has not previously been treated with an antidiabetic agent, e.g. metformin or a thiazolidinedione alone, for the treatment of type 2 diabetes.
- a combination of glutaminyl thiazolidine or a pharmaceutically acceptable salt thereof, and a thiazolidinedione for administration to a human who has not previously been treated with an antidiabetic agent, e.g. metformin or a thiazolidinedione alone, for the treatment of type 2 diabetes.

The DPIV inhibitor and the metformin or thiazolidinedione are each administered in a pharmaceutically acceptable form, including pharmaceutically acceptable derivatives such as pharmaceutically acceptable salts, esters and solvates thereof, as appropriate of the relevant pharmaceutically active agent. In certain instances herein the names used for the active agent may relate to a particular pharmaceutical form of the relevant active agent. It will be understood that the use of all pharmaceutically acceptable forms of the active agents per se is encompassed by this invention.

Pharmaceutically acceptable salts of the preferred DPIV inhibitor glutaminyl thiazolidine include acid addition salts, i.e. where the amino acid basic side chain is protonated with an inorganic or organic acid. Representative organic and inorganic acids include hydrochloric, hydrobromic, perchloric, sulfuric, nitric, phosphoric, acetic, propionic, glycolic, lactic, succinic, maleic, fumaric, malic, tartaric, citric, benzoic, mandelic, methanesulfonic, hydroxyethanesulfonic, benzenesulfonic, oxalic, pamoic, 2-naphthalenesulfonic, p-toulenesulfonic, cyclohexanesulfamic, salicylic, saccharinic, trifluoroacetic, sulfinic and 3,5-di-tert-butylbenzoic acid. Preferred acid addition salts include the fumarate, benzoate, maleinate, oxalate, 3,5-di-tertiary-butylbenzoate, salicylate, acetate and hydrochloride salts, especially the hydrochloride salt.

The DPIV inhibitor and the metformin or thiazolidinedione are preferably administered orally. In particular they are preferably formulated in unit doses for administration once, twice or three times a day. When the DPIV inhibitor is glutaminyl thiazolidine or a salt thereof it is preferably administered two or three times a day.

To prepare the pharmaceutical compositions of the invention the active agents are intimately admixed with a pharmaceutical carrier according to conventional pharmaceutical compounding techniques, which carrier may take a wide variety of forms depending of the form of preparation desired for administration, e.g. oral or parenteral such as intramuscular. In preparing compositions in oral dosage form, any of the usual pharmaceutical media may be employed. Thus, for liquid oral preparations, such as for example, suspensions, elixirs and solutions, suitable carriers and additives include water, glycols, oils, alcohols, flavoring agents, preservatives, coloring agents and the like; for solid oral preparations such as, for example, powders, capsules, gelcaps and tablets, suitable carriers and additives include starches, sugars, diluents, granulating agents, lubricants, binders, disintegrating agents and the like. Because of their ease in administration, tablets and capsules represent the most advantageous oral dosage unit form, in which case solid pharmaceutical carriers are obviously employed. If desired, tablets may be sugar coated or enteric coated by standard techniques. For parenterals, the carrier will usually comprise sterile water, through other ingredients, for example, for purposes such as aiding solubility or for preservation, may be included.

Injectable suspensions may also be prepared, in which case appropriate liquid carriers, suspending agents and the like may be employed. The pharmaceutical compositions herein will contain, per dosage unit, e.g. tablet, capsule, powder, injection, teaspoonful and the like, an amount of the active agent necessary to deliver a therapeutically effective amount. The dosages, however, may be varied depending upon the requirement of the patients, the severity of the condition being treated and the compound being employed. The use of either daily administration or post-periodic dosing may be employed.

Preferably these compositions are in unit dosage form such as tablets, pills, capsules, powders, granules, sterile parenteral solutions or suspensions, metered aerosol or liquid sprays, drops, ampoules, autoinjector devices or suppositories; for oral parenteral, intranasal, sublingual or rectal administration, or for administration by inhalation or insufflation. For preparing solid compositions such as tablets, the principal active agent is mixed with a pharmaceutical carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of the active agent(s). When referring to these preformulation compositions as homogeneous, it is meant that the active agent is dispersed evenly throughout the composition so that the composition may be readily subdivided into equally effective dosage forms such as tablets, pills and capsules. This solid preformulation composition is then subdivided into unit dosage forms of the type described above.

Tablets or pills can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids with such materials as shellac, cetyl alcohol and cellulose acetate.

Liquid forms in which the DPIV inhibitor and the metformin or thiazolidinedione may be incorporated for administration orally or by injection include, aqueous solutions, suitably flavoured syrups, aqueous or oil suspensions, and flavoured emulsions with edible oils such as cottonseed oil, sesame oil, coconut oil or peanut oil, as well as elixirs and similar pharmaceutical vehicles. Suitable dispersing or suspending agents for aqueous suspensions, include synthetic and natural gums such as tragacanth, acacia, alginate, dextran, sodium carboxymethylcellulose, methylcellulose, polyvinylpyrrolidone or gelatin and the like. Forms useful for parenteral administration include sterile solutions, emulsions and suspensions. Isotonic preparations which generally contain suitable preservatives are employed when intravenous administration is desired.

The method of treating type 2 diabetes as described herein may also be carried out using a pharmaceutical composition comprising a DPIV inhibitor, optionally in combination with metformin or a thiazolidinedione, and a pharmaceutically acceptable carrier. The pharmaceutical composition may be constituted into any form suitable for the mode of administration selected. Carriers include necessary and inert pharmaceutical excipients, including, but not limited to, binders, suspending agents, lubricants, flavorants, sweeteners, preservatives, dyes, and coatings. Compositions suitable for oral administration include solid forms, such as pills, tablets, caplets, capsules (each including immediate release, timed release and sustained release formulations), granules, and powders, and liquid forms, such as solutions, syrups, elixirs, emulsions, and suspensions. For instance, for oral administration in the form of a tablet or capsule, the active agents can be combined with an oral, non-toxic pharmaceutically acceptable inert carrier such as ethanol, glycerol, water and the like. Moreover, when desired or necessary, suitable binders; lubricants, disintegrating agents and coloring agents can also be incorporated into the mixture. Suitable binders include, without limitation, starch, gelatin, natural sugars such as glucose or betalactose, corn sweeteners, natural and synthetic gums such as acacia, tragacanth or sodium oleate, sodium stearate, magnesium stearate, sodium benzoate, sodium acetate, sodium chloride and the like. Disintegrators include, without limitation, starch, methyl cellulose, agar, bentonite, xanthan gum and the like.

The DPIV inhibitor and metformin or a thiazolidinedione may be administered in any of the foregoing compositions and according to dosage regimens whenever glycaemic control is required. Optimal dosages to be administered may be readily determined by those skilled in the art, and will vary with the particular active agent used, the mode of administration, the strength of the preparation, the mode of administration, and the advancement of disease condition. In addition, factors associated with the particular patient being treated, including patient age, weight, diet and time of administration, will result in the need to adjust dosages.

Suitable dosages, including especially unit dosages, of the active agents to be used in the method of the invention include the known dosages including unit doses for these compounds as described or referred to in reference text such as the British and US Pharmacopoeias, Remington's Pharmaceutical Sciences (Mack Publishing Co.), Martindale The Extra Pharmacopoeia (London, The Pharmaceutical Press) (for example see the 31st Edition page 341 and pages cited therein) or the above mentioned publications.

Suitable doses of DPIV inhibitors include those described in the relevant publications mentioned above. Suitable unit doses of the preferred DPIV inhibitor glutaminyl thiazolidine are in the range 10 to 500mg, e.g. 50, 100, 200 or 300mg, which unit doses may be administered up to three times a day.

A suitable daily dosage of metformin is between 50 and 3000mg, for example 250, 500mg, 850mg or 1000mg.

In one particular aspect the invention comprises administration of unit doses of 2 to 12 mg of 5-[4-[2-(N-methyl-N-(2-pyridyl)amino)ethoxy]benzyl]thiazolidine-2,4-dione.

Suitable unit dosages of TZDs include from 100 to 800mg of troglitazone such as 200, 400, 600 or 800mg or from 5 to 50mg, including 10 to 40mg, of pioglitazone, such as 20, 30 or 40 mg and also including 15, 30 and 45mg of pioglitazone.

Also, the dosages of each particular active agent in any given composition can as required vary within a range of doses known to be required in respect of accepted dosage regimens for that compound. Dosages of each active agent can also be adapted as required to take into account advantageous effects of combining the agents as mentioned herein.

The DPIV inhibitor and metformin or a thiazolidinedione may be taken before, during or after a meal. When taken before a meal they can be taken 1 hour, preferably 30 or even 15 or 5 minutes before eating. When taken after a meal they can be taken 5, 15 or 30 minutes or even 1 hour after finishing a meal.

Suitably, the particularly beneficial effect on glycaemic control in the treatment of type 2 diabetes provided by the combination of the invention is an improved therapeutic ratio relative to the therapeutic ratio for one compound of the combination when used alone and at a dose providing an equivalent efficacy to the combination of the invention.

Glycaemic control may be characterised using conventional methods, for example by measurement of a typically used index of glycaemic control such as fasting plasma glucose or glycosylated haemoglobin (HbAlc). Such indices are determined using standard methodology, for example those described in: Tuescher A, Richterich, P., Schweiz. med. Wschr. 101 (1971), 345 and 390 and Frank P., 'Monitoring the Diabetic Patent with Glycosolated Hemoglobin Measurements', Clinical Products 1988.

The dosage level of each of the active agents when used in accordance with the invention may be less than would have been required from a purely additive effect upon glycaemic control.

The term "therapeutically effective amount" as used herein, means that amount of active compound or pharmaceutical agent that elicits the biological or medicinal response in a tissue system, animal or human, being sought by a researcher, veterinarian, medical doctor or other clinician, which includes alleviation of the symptoms of the disease or disorder being treated.

## Claims

1. A DPIV inhibitor in combination with metformin or a thiazolidinedione for use as first-line therapy for the treatment of type 2 diabetes in a human, wherein the human has not previously been treated with an antidiabetic agent

2. A combination for use according to claim 1 wherein the human has failed to achieve adequate glycaemic control using diet, weight reduction and/or exercise alone.

3. A combination for use according to any one of the preceding claims wherein the DPIV inhibitor and metformin or a thiazolidinedione are administered simultaneously, sequentially or separately.

4. A combination for use according to any one of the preceding claims wherein the DPIV inhibitor and metformin or a thiazolidinedione are administered in a formulation which includes both the DPIV inhibitor and metformin or a thiazolidinedione.

5. A combination for use according to any one of the preceding claims wherein the DPIV inhibitor and metformin or a thiazolidinedione are administered orally.

6. A combination for use according to any one of the preceding claims wherein the DPIV inhibitor is glutaminyl thiazolidine or a pharmaceutically acceptable salt thereof.

7. A combination for use according to any one of claims I to 5 wherein the DPIV inhibitor is selected from sitagliptin, vildagliptin, saxagliptin, denagliptin and alogliptin and pharmaceutically acceptable salts thereof.

8. A combination for use according to any one of the preceding claims wherein the DPIV inhibitor and metformin or a thiazolidinedione are administered once, twice or three times a day.

9. A combination for use according to any one of the preceding claims wherein DPIV inhibitor is administered in combination with metformin.

10. A combination for use according to any one of claims 1 to 8 wherein the thiazolidinedione is troglitazone, ciglitazone, pioglitazone, englitazone or rosiglitazone.

11. A combination for use according to claim 10 wherein the thiazolidinedione is rosiglitazone.

## Patentansprüche

1. DPIV-Inhibitor in Kombination mit Metformin oder einem Thiazolidindion zur Verwendung als Initialtherapie zur Behandlung von Typ 2-Diabetes beim Menschen, wobei der Mensch vorher nicht mit einem antidiabetischen Mittel behandelt wurde.

2. Kombination zur Verwendung nach Anspruch 1, wobei es dem Menschen nicht gelungen ist, unter Verwendung einer Diät, Gewichtsreduktion und/oder Bewegung alleine eine adäquate glykämische Kontrolle zu erreichen.

3. Kombination zur Verwendung nach einem der vorangehenden Ansprüche, wobei der DPIV-Inhibitor und Metformin oder ein Thiazolidindion gleichzeitig, in Folge oder getrennt verabreicht werden.

4. Kombination zur Verwendung nach einem der vorangehenden Ansprüche, wobei der DPIV-Inhibitor und Metformin oder ein Thiazolidindion in einer Formulierung, die sowohl den DPIV-Inhibitor als auch Metformin oder ein Thiazolidindion enthält, verabreicht werden.

5. Kombination zur Verwendung nach einem der vorangehenden Ansprüche, wobei der DPIV-Inhibitor und Metformin oder ein Thiazolidindion oral verabreicht werden.

6. Kombination zur Verwendung nach einem der vorangehenden Ansprüche, wobei der DPIV-Inhibitor Glutaminylthiazolidin oder ein pharmazeutisch annehmbares Salz davon ist.

7. Kombination zur Verwendung nach einem der Ansprüche 1 bis 5, wobei der DPIV-Inhibitor ausgewählt wird aus Sitagliptin, Vildagliptin, Saxagliptin, Denagliptin und Alogliptin und pharmazeutisch annehmbaren Salzen davon.

8. Kombination zur Verwendung nach einem der vorangehenden Ansprüche, wobei der DPIV-Inhibitor und Metformin oder ein Thiazolidindion einmal, zweimal oder dreimal täglich verabreicht werden.

9. Kombination zur Verwendung nach einem der vorangehenden Ansprüche, wobei der DPIV-Inhibitor in Kombination mit Metformin verabreicht wird.

10. Kombination zur Verwendung nach einem der Ansprüche 1 bis 8, wobei das Thiazolidindion Troglitazon, Ciglitazon, Pioglitazon, Englitazon oder Rosiglitazon ist.

11. Kombination zur Verwendung nach Anspruch 10, wobei das Thiazolidindion Rosiglitazon ist.

## Revendications

1. Inhibiteur de DPIV en combinaison avec de la metformine ou une thiazolidinedione, pour utilisation en tant que thérapie de première intention pour le traitement du diabète de type 2 chez un humain, dans lequel l'humain n'a auparavant pas été traité avec un agent antidiabétique.

2. Combinaison pour utilisation selon la revendication 1, dans laquelle l'humain a échoué à atteindre un contrôle de glycémie adéquat par un régime alimentaire, une perte de poids et/ou de l'exercice uniquement.

3. Combinaison pour utilisation selon l'une quelconque des revendications précédentes, dans laquelle l'inhibiteur de DPIV et la metformine ou une thiazolidinedione sont administrés simultanément, successivement ou séparément.

4. Combinaison pour utilisation selon l'une quelconque des revendications précédentes, dans laquelle l'inhibiteur de DPIV et la metformine ou une thiazolidinedione sont administrés dans une formulation qui contient à la fois l'inhibiteur de DPIV et la metformine ou une thiazolidinedione.

5. Combinaison pour utilisation selon l'une quelconque des revendications précédentes, dans laquelle l'inhibiteur de DPIV et la metformine ou une thiazolidinedione sont administrés par voie orale.

6. Combinaison pour utilisation selon l'une quelconque des revendications précédentes, dans laquelle l'inhibiteur de DPIV est la glutaminyl-thiazolidine ou un sel pharmaceutiquement acceptable de celle-ci.

7. Combinaison pour utilisation selon l'une quelconque des revendications 1 à 5, dans laquelle l'inhibiteur de DPIV est choisi parmi la sitagliptine, la vildagliptine, la saxagliptine, la dénagliptine et l'alogliptine, ainsi que leurs sels pharmaceutiquement acceptables.

8. Combinaison pour utilisation selon l'une quelconque des revendications précédentes, dans laquelle l'inhibiteur de DPIV et la metformine ou une thiazolidinedione sont administrés une, deux ou trois fois par jour.

9. Combinaison pour utilisation selon l'une quelconque des revendications précédentes, dans laquelle l'inhibiteur de DPIV est administré en combinaison avec de la metformine.

10. Combinaison pour utilisation selon l'une quelconque des revendications 1 à 8, dans laquelle la thiazolidine-dione est la troglitazone, la ciglitazone, la pioglitazone, l'englitazone ou la rosiglitazone.

11. Combinaison pour utilisation selon la revendication 10, dans laquelle la thiazolidinedione est la rosiglitazone.
